# EUROPEAN PATENT SPECIFICATION

(11) **EP 3 290 527 B1**
(45) Date of publication and mention of the grant of the patent: **19.06.2019**
(21) Application number: 16186104.2
(22) Date of filing: 29.08.2016
(51) Int. Cl.: C12Q 1/6841, C12Q 1/689

(54) **METHOD FOR DETECTING MICROORGANISMS IN A SAMPLE**
VERFAHREN ZUM NACHWEIS VON MIKROORGANISMEN IN EINER PROBE
PROCÉDÉ DE DÉTECTION DE MICRO-ORGANISMES DANS UN ÉCHANTILLON

(43) Date of publication of application: 07.03.2018
(73) Proprietor: MetaSystems Indigo GmbH, 68804 Altlußheim (DE)
(72) Inventor: VON STEIN, Walter, 40474 Düsseldorf (DE); STANGE, Mirko, 40213 Düsseldorf (DE)
(74) Representative: Remus, Alvaro Johannes

(56) References cited:
- WO-A1-2012/035076
- WO-A2-2008/043543
- XI C ET AL: "Use of DNA and peptide nucleic acid molecular beacons for detection and quantification of rRNA in solution and in whole cells", APPLIED AND ENVIRONMENTAL MICROBIOLOGY, AMERICAN SOCIETY FOR MICROBIOLOGY, US, vol. 69, no. 9, 1 September 2003 (2003-09-01), pages 5673-5678, XP002354418, ISSN: 0099-2240, DOI: 10.1128/AEM.69.9.5673-5678.2003
- SHINSUKE SANDO ET AL: "Quenched Auto-Ligating DNAs: Multicolor Identification of Nucleic Acids at Single Nucleotide Resolution", JOURNAL OF THE AMERICAN CHEMICAL SOCIETY, vol. 126, no. 4, 1 February 2004 (2004-02-01), pages 1081-1087, XP055214551, ISSN: 0002-7863, DOI: 10.1021/ja038665z
- CHRISTINA SAKARIKOU ET AL: "Beacon-based (bbFISH?) technology for rapid pathogens identification in blood cultures", BMC MICROBIOLOGY, BIOMED CENTRAL LTD, GB, vol. 14, no. 1, 22 April 2014 (2014-04-22) , page 99, XP021181471, ISSN: 1471-2180, DOI: 10.1186/1471-2180-14-99
- CHUANWU XI ET AL: "Use of molecular beacons for the detection of bacteria in microfluidic devices", SPIE - INTERNATIONAL SOCIETY FOR OPTICAL ENGINEERING. PROCEEDINGS, vol. 4982, 25 January 2003 (2003-01-25), page 170, XP055345931, US ISSN: 0277-786X, DOI: 10.1117/12.478143 ISBN: 978-1-5106-0753-8
- KONCAN R ET AL: "Direct identification of major Gram-negative pathogens in respiratory specimens by respiFISH(R) HAP Gram (-) Panel, a beacon-based FISH methodology", EUROPEAN JOURNAL OF CLINICAL MICROBIOLOGY & INFECTIOUS DISEASES, SPRINGER, WIESBADEN, DE, vol. 34, no. 10, 29 July 2015 (2015-07-29) , pages 2097-2102, XP035544987, ISSN: 0934-9723, DOI: 10.1007/S10096-015-2458-Y [retrieved on 2015-07-29]

## Description

### Background of the invention

The invention relates to a method for detecting at least one specific microorganism in a sample, wherein at least one probe nucleic acid sequence is capable of hybridizing with at least one target nucleic acid sequence of said microorganism, said probe nucleic acid sequence comprising at least one detectable label which is capable of emitting at least one detectable signal, wherein the detectable signal takes a first value when the probe sequence is not bound to the target sequence and a second value when the probe sequence is bound to the target sequence, wherein the second value of the detectable signal is decreased, increased or changed compared to the first value of the detectable signal.

### Prior art

Fast and reliable detection of microorganisms (e.g. pathogen bacteria) in biological samples such as blood, sputum and other secretions still remains a problem in human health care, in particular in hospitals. It is however of major importance to rapidly diagnose an infectious disease at the point of care so as to quickly initiate or adapt antibiotic treatment and thus improve patient care. Fast diagnosis of critical infectious diseases also contributes to substantial cost savings due to shorter hospitalization times.

For example, community-acquired pneumonia (CAP) remains a major cause of morbidity and mortality worldwide. Streptococcus pneumoniae, Mycoplasma pneumoniae, influenza A virus (InfA), respiratory syncytial virus (RSV), and adenoviruses (AdV) amongst others are recognized as important causes of CAP. Despite efforts to find evidence for bacterial and viral pathogens as etiological agents in patients with CAP, etiology remains elusive in up to 50% of the patients, compromising effective treatment. Differences in the causative pathogens are observed between countries and health care settings, but also differences in methods used to identify the causative agent contribute to the diverse numbers of prevalence. The lack of sensitive methods to identify the pathogen adds to the problem.

In hospital-acquired pneumonia there is a great need for a Point-of-Care diagnostic device (POC) since there are no good alternatives to classical identification methods. The gold standard is culture, which does not show very good sensitivity and is time consuming. It is desirable to get an accurate, up-to-date picture of the actual situation of the patient, especially in intensive care units. It is known that the problem causing bacterium can shift rather rapidly and treatment decisions have to be made based on the current situation of the patient.

Automated PCR-based diagnostic tools such as Unyvero® (Curetis AG, Holzgerlingen, Germany) or the FilmArray® System (Biofire Diagnostics, Salt Lake City, USA) require high cap-ex investment, are very cost-intensive, are limited to sample materials needed (e.g.: swabs only), and are therefore not likely to be used in routine diagnostics.

Sakarikou et al (2014, BMC Microbiology, vol.14, p.99) described rapid identification of pathogens in blood cultures using beacon-based FISH technology.

Xi et al (2003, Proceedings of SPIE, vol.4982, p.170) described the use of molecular beacons for the detection of bacteria in microfluidic devices.

### Summary of the invention

It is the object of the invention to provide a fast and low-cost method for detecting specific microorganisms in a sample, which allows for non-invasive detection of the microorganisms directly from a large variety of sample materials such as blood cultures or sputa without the need for amplification or cultivation. The object is achieved by a method as initially specified, comprising:
a) in a provided sample suspected to include the microorganism;
b) perforating or lysing microorganisms within the sample;
c) adding the probe nucleic acid sequence to the sample under conditions which allow *in-vitro* hybridization of the probe sequence with the target sequence, wherein said conditions comprise heating the sample at least temporarily to a predetermined temperature above room temperature and then cooling the sample to a lower temperature;
d) continuously measuring values of the detectable signal for the probe nucleic acid sequence in the sample at a plurality of measuring points in time along the course of the signal-generating reaction, comprising measuring values of the detectable signal during and/or after heating and during and/or after cooling; and
e) continuously analyzing the measured values of the detectable signal of the probe nucleic acid sequence, wherein it is indicated that the sample contains the microorganism if the measured values correspond to the second value of the detectable signal.

The method according to the invention provides an advantageous solution that can non-invasively detect and identify specific microorganisms directly in samples (i.e. a defined volume of an aqueous solution or suspension), for example respiratory samples, without the need for amplification or cultivation. For example, using fluorescently labeled probes, the method according to the invention cannot only differentiate and identify a plurality of bacterial strains in one simple multiplexed test, but also exclude a bacterial infection by the use of a universal probe which acts as a positive control. The entire test procedure only takes about 15 minutes to complete and the analytical sensitivity is very high (detection limit of about 10⁴ - 10⁶ cfu/ml, cfu = colony-forming units). The method according to the invention is simple and cost effective, and does not require highly skilled personnel. Moreover, the method according to the invention may open up the possibility to perform semi-quantification of the bacterial load, at least at the higher end of the detection limit, of a plurality (e.g. up to 20) different bacterial strains allowing the monitoring of patients at the point of care.

The *in-vitro* hybridization in step c) may be performed at a temperature between about 30°C and about 65°C, in particular between about 35°C and about 59°C. Especially, the temperature may be about 52°C. "Heating" or "raising the temperature of the sample to a temperature above room temperature" as used herein refers to a process wherein thermal energy is transferred to the sample so as to increase its temperature from about 20-25°C (room or ambient temperature) to a temperature above 30°C. Such process can be accomplished, for example, using a heating device (e.g., a radiator block) that is capable of transferring heat to a container including the sample.

The incubation time of the *in-vitro* hybridization in step c) may be between about 1 and about 30 minutes, in particular up to 15 minutes or up to 10 minutes, especially about 15 minutes or about 10 minutes. The preferred length of incubation is between about 2 and about 6 minutes. It is more preferred to incubate about 4 minutes.

Both the measurement of the value of the detectable signal in the sample in step d) and the analysis of the measured value in step e) are accomplished continuously, wherein the measurement may be stopped once the analysis in step e) indicates that the sample contains the microorganism. Detection of the microorganism may be indicated, for example, at some point during the course of the analyzed signal-generating reaction or at the end of the analyzed reaction. In any case, continuous measurement as used herein refers to a real-time determination of the value of the detectable label at a plurality of measuring points along the course of the analyzed signal-generating reaction.

In an exemplary and advantageous embodiment of the invention at least steps d) and e) are conducted automatically, and the measured value is read out electronically and processed digitally. For example, the method according to the invention is suitable to be accomplished by an automated reader system such as, for example, the ESEQuant Tube Scanner (Qiagen, Hilden, Germany) or the AXXIN T 16-ISO fluorescence detection system (AXXIN, Fairfield, Australia). Accordingly, the method according to the invention is well-suited for automation and can be performed without highly skilled personnel. Automation makes the diagnostic method not only easy but also very fast. For example, after preparation of the sample, which may include liquefying and/or diluting the sample with a lysis buffer and only takes about 1 minute, readout of the results from the automated reader system can be accomplished within only 15 minutes. Accordingly, detection and identification of specific microorganisms can be performed very fast at the point of care so that effective treatment of the patient can be initiated in an early stage of infection.

In another exemplary and advantageous embodiment of the invention the temperature is kept constant after step c) for a predetermined period of time (incubation period) so as to ascertain that as many probe nucleic acid sequences as possible are ready to hybridize with the target nucleic acid sequence. In order to ensure accuracy and reliability of the analysis, at least one value of the detectable signal in the sample may be measured while the temperature is kept constant.

"Keeping the temperature constant" as used herein refers to a process of controlling the temperature of the sample such that its temperature does not vary more than about 1°C or 2°C within a certain period of time.

Accuracy and reliability of the analysis are increased by measuring the signal during or after the cooling period since signals emitted from unbound probes are eliminated or at least decreased at lower temperatures, at least when molecular beacon probes are used.

"Cooling" as used herein refers to a process wherein thermal energy is withdrawn from the sample so as to decrease its temperature from a temperature above 30°C to a lower temperature, in particular about 20-25°C. Such process can be accomplished, for example, using a cooling device (e.g., a radiator block) that is capable of withdrawing heat from a container including the sample.

Basically, measuring one value of the detectable signal at any point in time during the entire process should be sufficient to obtain a reliable result. However, measuring continuously the detectable signal in the sample further enhances accuracy of the method. Moreover, this makes it possible to stop measuring before the analyzed signal-generating reaction has been finished completely.

For example, the probe nucleic acid sequence may be a linear nucleic acid or a molecular beacon. The probe nucleic acid sequence may in particular be an oligonucleotide capable of specifically hybridizing with the target nucleic acid sequence in the micro-organism under *in-vitro* conditions. The skilled person knows suitable conditions under which the probe nucleic acid sequence selectively hybridizes with the target sequence. The oligonucleotide may have a length of up to 50 nucleotides, for example from 10 to 50 nucleotides. The oligonucleotide may be a linear oligonucleotide. Alternatively, the oligonucleotide may be a molecular beacon, as for example described in WO 2008/043543 A2.

Suitable conditions for hybridization of molecular beacons are exemplarily described in WO 2008/043543 A2 as well.

A molecular beacon, as used herein, can be a nucleic acid capable of forming a hybrid with the target nucleic acid sequence and capable of forming a stem-loop structure if no hybrid is formed with the target sequence, said nucleic acid may comprise:
- a nucleic acid portion comprising a sequence (a1) complementary to the target nucleic acid sequence, and a pair of two complementary sequences (a2) capable of forming a stem and flanking the sequence (a1), and
- an effector and an inhibitor, wherein the inhibitor inhibits the effector when the nucleic acid forms a stem-loop structure, and wherein the effector is active when the nucleic acid is not forming a stem-loop structure.

A molecular beacon can also be termed as "beacon", "hairpin", or "hairpin loop", wherein the "open" form (no stem is formed) as well as the "closed" form (the beacon forms a stem) are included. The open form includes a beacon not forming a hybrid with a target sequence and a beacon forming a hybrid with the target sequence. Details of molecular beacons are disclosed in WO 2008/043543 A2.

In another exemplary and advantageous embodiment of the invention the target nucleic acid sequence is a DNA sequence or an RNA sequence, in particular an rRNA sequence. For example, with the method according to the invention about 10⁴ - 10⁶ cfu/ml, at least 10⁶ - 10⁸ cfu/ml, can be reliably detected by hybridization of the probe sequence with rRNA without prior amplification.

In another exemplary and advantageous embodiment of the invention additionally at least one first control nucleic acid sequence is added to the sample in step c), values of a detectable signal for the first control nucleic acid sequence being measured at the measuring points in step d), and the analysis in step e) comprises a comparison of the measured values of the detectable signal of the probe nucleic acid sequence with the measured values of the detectable signal of the first control nucleic acid sequence, wherein it is indicated that the sample contains the microorganism if the value measured for the probe nucleic acid sequence at any measuring point is different from the respective value measured for the first control nucleic acid sequence and the difference between the compared values exceeds a predetermined threshold. The first control sequence is a nucleic acid sequence which is not complementary and thus cannot bind to any potential nucleic acid target sequence of microorganisms. The use of a first control nucleic acid sequence that does bind to any target sequence of the microorganism as a reference for the analysis of the signal's value further enhances accuracy and reliability of the method according to the invention.

The first control nucleic acid sequence can be, for example, a sequence that does not bind to any nucleic acid sequence. Such nonsense sequence represents a suitable negative control for validating the test results, i.e. no signal is expected for this control sequence.

Moreover, at least one second control nucleic acid sequence that binds to nucleic acid sequences of all microorganisms can be additionally added to the sample in step c). Such universal sequence represents a suitable positive control for validating the test results, with which a known signal can be expected. Such positive control may also be used to exclude that the patient is infected with a microorganism at all.

In another exemplary and advantageous embodiment of the invention the sample is liquefied and/or diluted after step a) and/or during step b). If the sample is a viscous respiratory sample (e.g. sputum), it is important to liquefy and even dilute the sample in order to render it suitable for hybridization step c). For example, a dilution ratio between 1.0:0.5 and 1.0:5.0, in particular 1:1 or 1:2, can be suitable for whole blood samples or sputum samples. In an exemplary embodiment the sample is liquefied by addition of a specific aqueous buffer solution at a ratio of 1:1.

In step b) of the method according to the invention, the microorganisms can be perforated or lysed, for example, by enzymatic, mechanical, ultrasonic and/or chemical treatment, in order to obtain reproducible and reliable results. To this end, enzymatic lysis is often the most effective option, even because enzymes usually do not impair the following hybridization process. However, it is particularly important that the cells are either perforated enough so that the probes can enter the cells for the hybridization reaction or that the cells are lysed and the rRNA is released.

In another exemplary and advantageous embodiment of the invention the detectable label is a luminescent label, in particular a fluorescent label. For example, the method according to the invention may be based on the well-known fluorescence *in-situ* hybridization (FISH) technology. To this end, the probe nucleic acid sequence may be coupled with a suitable fluorophore, for example, non-protein organic compounds such as ATTO (ATTO-TEC GmbH, Siegen, Germany), FAM™ (fluorescein), or HEX™. Each multiplex test may be conducted, for example, on an 8- or 12-field strip or an 8- or 12-chamber cartridge, of which the first field/chamber is the positive control. Of course, 96-well or 384-well plates, or any other suitable format, can be used as well to accomplish the method according to the invention. In most cases this is sufficient due to the fact that the sample material is known, and therefore the number of clinically relevant bacteria is limited. All tests can be designed to detect at least 90% of relevant bacteria.

In an alternative exemplary and advantageous embodiment of the invention the detectable label is a reporter enzyme, preferably selected from the group consisting of tyrosinase, peroxidase, sulfite oxidase, alkaline phosphatase, glucose oxydase, guanine oxidase.

In another exemplary and advantageous embodiment of the invention the sample is at least one sample selected from the group consisting of whole blood, blood culture, liquor, sputum and other secretions, human and/or veterinarian clinical samples, and industrial samples such as food, beverages, and water. Accordingly, the method according to the invention is useful in several applications such as diagnostics for most critical infectious diseases in hospitals and laboratories, testing of food and beverages in industrial production, water testing, or veterinarian testing.

Also disclosed herein is a composition for detecting at least one specific microorganism in a sample, said composition comprising:
a) at least one perforation or lysis reagent for perforating or lysing the microorganisms;
b) at least one probe nucleic acid sequence being capable of hybridizing with at least one target nucleic acid sequence of said microorganism, said probe nucleic acid sequence comprising at lest one detectable label which is capable of emitting at least one detectable signal, wherein the detectable signal takes a first value when the probe sequence is not bound to the target sequence and a second value when the probe sequence is bound to the target sequence, wherein the second value of the detectable signal is decreased, increased or changed compared to the first value of the detectable signal; and
c) at least one buffer and/or enhancer substance for adjusting conditions which allow *in-vitro* hybridization of the probe sequence with the target sequence.

With this composition an all-in-one, ready-to-use solution is provided, which enables the user of said composition to perform a fast and easy diagnostic test without specific skills and intensive training. The method according to the invention can be easily accomplished with said composition which is also well-suited for automated processes. In particular, the perforation or lysis reagent should include a component that enables (complete) lysis or at least perforation of the microorganisms in the sample, for example, a lytic enzyme such as lysozyme. A common hybridization buffer may be used, which can comprise, e.g., a suitable buffer substance such as NaH₂P0₄, formamide, a surfactant such as sodium dodecyl sulfate (SDS) and/or at least one salt such as NaCl. For fast analysis, the probe nucleic acid sequence should be coupled with a suitable label such as a fluorescent group that can be easily detected.

Optionally, the composition can additionally comprise a control nucleic acid sequence which is coupled with a detectable label that can be easily distinguished from the label of the probe sequence.

In the composition, the probe nucleic acid sequence may be a linear nucleic acid or a molecular beacon. The probe nucleic acid sequence may in particular be an oligonucleotide capable of specifically hybridising with a target nucleic acid sequence in the micro-organism under *in-vitro* conditions. The oligonucleotide may be a linear oligonucleotide or a molecular beacon such as the one described in WO 2008/043543 A2. For example, the detectable label which is coupled to the oligonucleotide is a luminescent label, in particular a fluorescent label. The probe nucleic acid sequence may be coupled with a suitable fluorophore, for example, a non-protein organic compound such as ATTO (ATTO-TEC GmbH, Siegen, Germany), FAM™ (fluorescein), or HEX™.

The lysis reagent in the composition may be an enzyme which ensures (complete) lysis or at least perforation of the cells and does not impair hybridization of the probe sequence with the target sequence.

Further disclosed herein is a kit for conducting the method according to the invention as described above, wherein said kit comprises the composition as described above.

Further disclosed herein is the use of a kit for detecting at least one specific microorganism in a sample in the method according to the invention. The invention is further described in detail with reference to the figures.

### Brief description of the figures

**Figure 1** shows sample read-outs taken with a fluorescent microscope using 1000x magnification, in particular read-outs from plates, from positive blood culture bottles (BCBs), and directly from respiratory samples. The samples were incubated with molecular beacons labelled with fluorophores.
   A - Klebsiella pneumoniae from culture,
   B - Escherichia coli from positive blood culture bottle (BCB), and
   C - Streptococcus pneumoniae from sputum.
**Figure 2** shows a graphical representation of probe sequence kinetics (1) without and (2) with target (fluorescence signal intensity [mV] over time (t) [min]). Within the first 4 minutes of the protocol the tube was heated up to 52°C, then incubated for 5 minutes and cooled down to RT again. During the whole course, the fluorescence was measured. The gray areas referred to as Δ1-Δ3 represent potential measuring points.
**Figure 3** shows a graphical representation of probe sequence kinetics with and without target (fluorescence signal intensity [mV] over time (t) [min], label = FAM);
   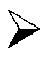 Tubes 1 and 4 (lower graphs): Probe sequences specific to E. coli (5 pmol) without target (Tube 1) or with "false target" (Tube 4: S. aureus);
   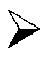 Tube 2 (upper graph): Probe sequence (E. coli, 5 pmol) with target (E. coli), amount of target high (15 pmol);
   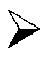 Tube 3 (middle graph): Probe sequence (E. coli, 5 pmol) with target (E. coli), amount of target low (7 pmol).

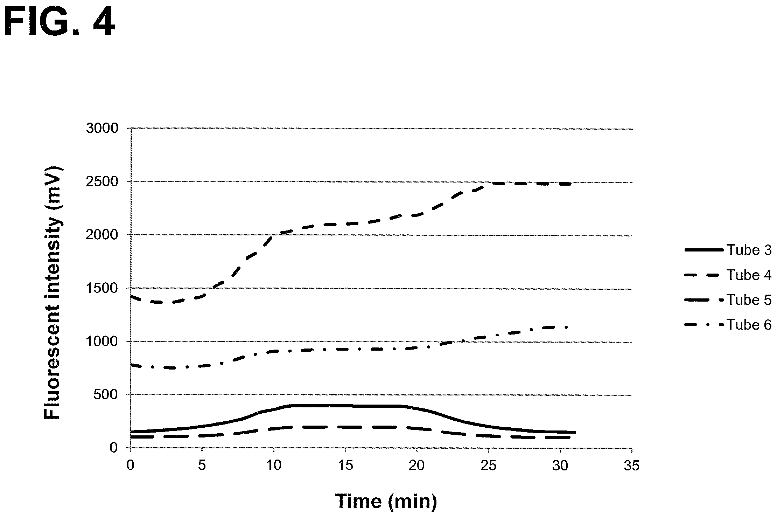

**Figure 4** shows a graphical representation of probe sequence kinetics with and without target (fluorescence signal intensity [mV] over time (t) [min], label = FAM);
   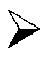 Tubes 3 and 5 (lower graphs): Probe sequences without target;
   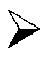 Tubes 4 and 6 (upper graphs): Probe sequences with target.

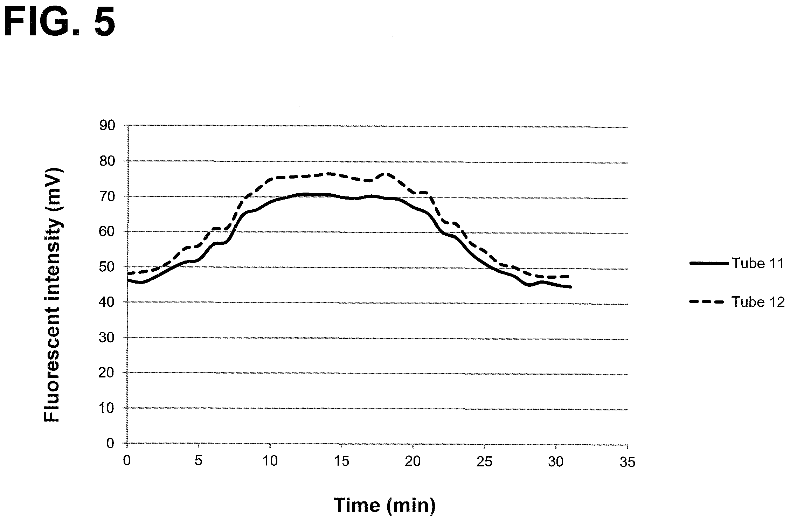

**Figure 5** shows a graphical representation of probe sequence kinetics without target but with different labels (HEX instead of FAM), fluorescence signal intensity [mV] over time (t) [min]; Tubes 11 and 12.

### Exemplary and preferred embodiments of the invention

The method according to the invention can be based, for example, on the well-known fluorescence in-situ hybridization (FISH) technology, which is improved by e.g. eliminating error-prone washing steps or reducing hybridization times dramatically down to less than 10 minutes. These improvements are achieved, among other factors, by using molecular beacons and a newly developed way to design the probe sequences used (as described in WO 2008/043543 A2).

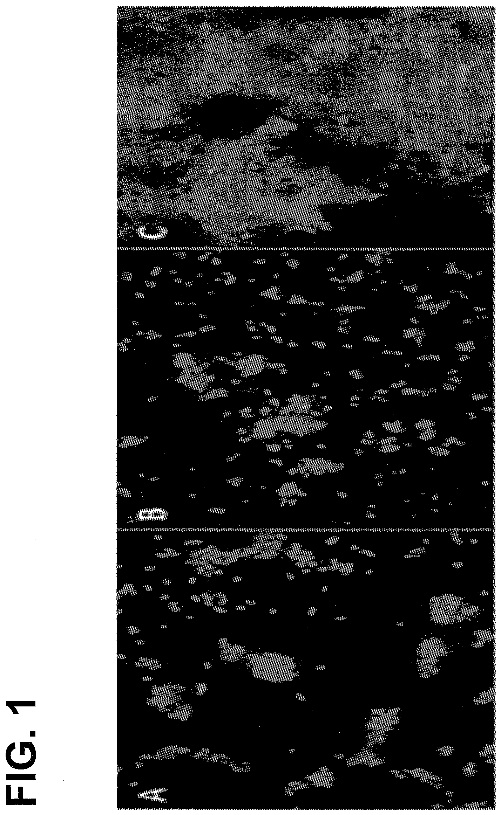

Sample read-outs taken with a fluorescent microscope using 1000x magnification are shown in **Figure 1**. The stained bacteria show a high and robust strong signal to noise ratio, regardless whether the material is taken from culture (Figure 1A), positive blood culture bottles (Figure 1B) or directly from respiratory secretions (Figure 1C). The intensity and clarity of the signal allows an automated read-out, even in the case of respiratory samples.

For example, Qiagen's ESEQuant tube scanner can be used as a tool for automation of the method according to the invention. This device is a small easy-to-use fluorescent measurement system that is extremely sensitive, robust and cost effective. However, alternatives exist if required. In a first exemplary approach the ESEQuant TS2 System was used to perform the method according to the invention. The conditions of the respective experiments are shown in Tables 1 and 2.

In a first step the opening and closing of the beacon itself was monitored and a low intensity at room temperature was expected since the beacon is in its closed conformation not emitting a signal. Upon heating, the beacon opens up, quencher and fluorophore are separated and light is emitted. Upon cooling, the beacon returned to its closed confirmation, which can be seen by a decreasing fluorescent signal. It is noted that for these experiments the sensitivity of the tube scanner was set to its minimum and a standard beacon concentration was used. An increase in sensitivity of the scanner and beacon concentration should also increase the signal intensity for later applications. It is also shown that there is a clear difference between presence and absence of the probe-specific target: In case the specific target is present, the signal stays at a higher level. Without any target present, the signal comes back to the ground line.

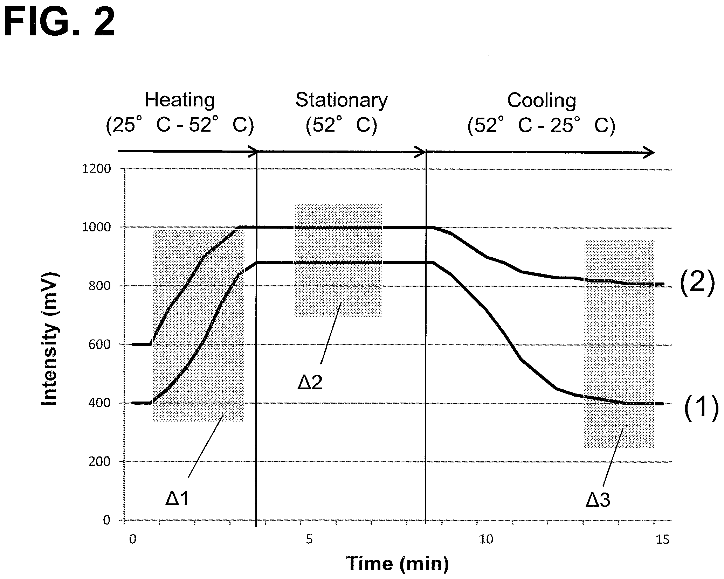

**Figure 2** (signal (1), probe without target) shows that, in the absence of target molecules, beacons open up during the heating phase and emit a fluorescent signal. During the stationary phase, beacons are in an equilibrium of open and closed conformation "looking" for their targets. The fluorescent signal remains constant in this phase. Upon cooling, the beacons return to their original closed conformation and the fluorescent signal ceases. Figure 2 (signal (2), probe with target) further shows that, already during the heating phase, probes open up rapidly in the presence of target molecules (Δ1). During the stationary phase equilibrium is shifted. That is, large amounts of probes are bound to target sequences while the remainder of probes is still in a 50% equilibrium (Δ2). After cooling, probes bound to target molecules remain open and still emit light (Δ3).

Figure 2 shows that there is a clear and robust difference between a probe sequence that hybridizes with a target sequence (signal (2)) and a control sequence which does not bind to any target (signal (1)). At any time during the entire hybridization process, i.e. throughout all phases (heating, stationary and cooling), the value of the detectable signal (2) is higher than the value of the control signal (1). However, the most significant difference can be observed at the end of the cooling phase (Δ3) so that measuring and analyzing the detectable signal (2) during this period provides the most reliable result. Optionally, additional measuring points in other phases (Δ1 and/or Δ2) can be set in order to verify the result and provide more certainty and reliability in respect of the final conclusion. The threshold for determining whether a microorganism is present may be either a certain value of the probe signal (e.g., fluorescence intensity) or a predetermined difference between the signals of the probe sequence and a suitable control sequence. In any case, the method according to the invention allows for fast and reliable detection of microorganisms in a sample.

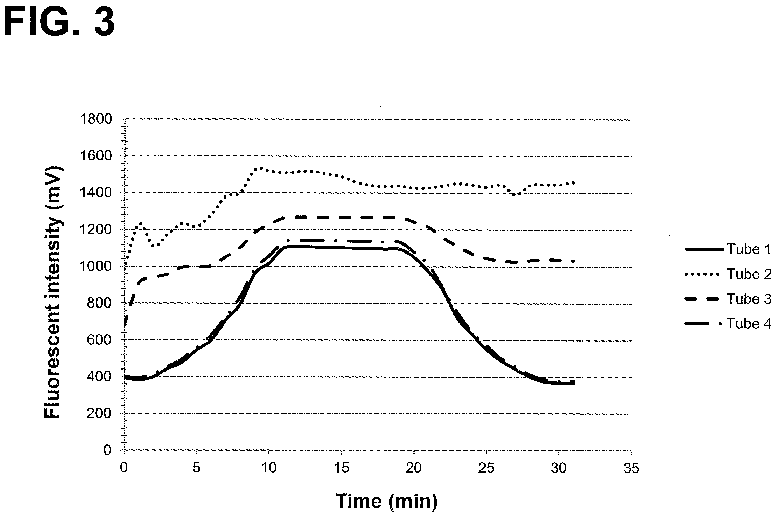

**Figure 3** **(Table 1**) again shows that beacon probes without target open during heating and close during cooling (Tubes 1 and 4). Addition of target sequences keeps the probe sequences open (Tubes 2 and 3), wherein more target (higher target concentration) increases the detectable signal (Tube 2) compared to samples with lower target concentration (Tube 3).

**Figure 4** **(Table 2**) shows that the detectable signal with samples including target sequences runs as a flat curve (Tubes 3 and 5), while a clear detectable signal with higher intensity can be observed with samples including target sequences (Tubes 4 and 6).

**Figure 5** **(Table 2**) shows that probe sequences labeled with a different fluorophore (HEX instead of FAM) are clearly detectable as well (Tubes 11 and 12).

As a result, the experimental results show that specific fluorescent signals can be detected, pathogen/probe specific signals can be differentiated, opening and closing of probes can be monitored in real time, and the signal to noise ratio is sufficient for automated analysis.

**Table 1: Run 2 (52 °C - 5 min., 30 °C - 2 min.)**

| **Tube** | **Probe (50 µl)** | **Target (10 µl**) | **Helper** | **RNA (10 µl)** |
|---|---|---|---|---|
| 1 | E.coli 5 pmol | / | / | / |
| 2 | E.coli 5 pmol | E.coli 15 pmol | / | / |
| 3 | E.coli 5 pmol | E.coli 7 pmol | / | / |
| 4 | E.coli 5 pmol | / | / | 50 ng |
| 5 | E.coli 5 pmol | / | / | 200 ng |
| 6 | S.aureus 5 pmol | / | / | / |
| 7 | S.aureus 5 pmol | S.aureus 10 pmol | / | / |
| 8 | S.aureus 5 pmol | S.aureus 5 pmol | / | / |
| 9 | E. faecium 5 pmol | / | / | / |
| 10 | E. faecium 5 pmol | E. faecium 30 pmol | / | / |
| 11 | E. faecium 5 pmol | E. faecium 15 pmol | / | / |

**Table 2: Run 3 (52 °C - 5 min., 30 °C - 2 min.)**

| **Tube** | **Probe (50 µl)** | **Target (10 µl)** | **Helper** | **RNA (10 µl)** |
|---|---|---|---|---|
| 1 | E.coli 5 pmol | / | / | / |
| 2 | E.coli 5 pmol | E.coli 15 pmol | / | / |
| 3 | E. faecium 5 pmol | / | / | / |
| 4 | E. faecium 5 pmol | E. faecium 15 pmol | / | / |
| 5 | E. faecium 5 pmol | / | Yes | / |
| 6 | E. faecium 5 pmol | E. faecium 15 pmol | Yes | / |
| 7 | E. faecium ATTO | / | Yes | / |
| 8 | E. faecium ATTO | / | Yes | / |
| 9 | E. faecium ATTO | E. faecium 15 pmol | Yes | / |
| 10 | E. faecium ATTO | E. faecium 15 pmol | Yes | / |
| 11 | Poscon Hex | / | Yes | / |
| 12 | Poscon Hex | / | Yes | / |

## Claims

1. Method for detecting at least one specific microorganism in a sample, wherein at least one probe nucleic acid sequence is capable of hybridizing with at least one target nucleic acid sequence of said microorganism, said probe nucleic acid sequence comprising at least one detectable label which is capable of emitting at least one detectable signal, wherein the detectable signal takes a first value when the probe sequence is not bound to the target sequence and a second value when the probe sequence is bound to the target sequence, wherein the second value of the detectable signal is decreased, increased or changed compared to the first value of the detectable signal, said method comprising:
a) in a provided sample suspected to include the microorganism;
b) perforating or lysing microorganisms within the sample;
c) adding the probe nucleic acid sequence to the sample under conditions which allow *in-vitro* hybridization of the probe sequence with the target sequence, wherein said conditions comprise heating the sample at least temporarily to a predetermined temperature above room temperature and then cooling the sample to a lower temperature;
d) continuously measuring values of the detectable signal for the probe nucleic acid sequence in the sample at a plurality of measuring points in time along the course of the signal-generating reaction, comprising measuring values of the detectable signal during and/or after heating and during and/or after cooling; and
e) continuously analyzing the measured values of the detectable signal of the probe nucleic acid sequence, wherein it is indicated that the sample contains the microorganism if the measured values correspond to the second value of the detectable signal.

2. Method according to claim 1, wherein the measurement is stopped once the analysis in step e) indicates that the sample contains the microorganism.

3. Method according to claim 1 or 2, wherein at least steps d) and e) are conducted automatically, and the measured values are read out electronically and processed digitally.

4. Method according to any one of claims 1 to 3, wherein after step c) the temperature is kept constant for a predetermined period of time.

5. Method according to claim 4, wherein at least one value of the detectable signal in the sample is measured while the temperature is kept constant.

6. Method according to any one of claims 1 to 5, wherein the probe nucleic acid sequence is a linear nucleic acid or a molecular beacon.

7. Method according to any one of claims 1 to 6, wherein the target nucleic acid sequence is a DNA sequence or an RNA sequence, in particular an rRNA sequence.

8. Method according to any one of claims 1 to 7, wherein in step c) additionally at least one first control nucleic acid sequence is added to the sample, values of a detectable signal for the first control nucleic acid sequence being measured at the measuring points in step d), and wherein the analysis in step e) comprises a comparison of the measured values of the detectable signal of the probe nucleic acid sequence with the measured values of the detectable signal of the first control nucleic acid sequence, wherein it is indicated that the sample contains the microorganism if the value measured for the probe nucleic acid sequence at any measuring point is different from the respective value measured for the first control nucleic acid sequence and the difference between the compared values exceeds a predetermined threshold.

9. Method according to claim 8, wherein the first control nucleic acid sequence is a sequence that does not bind to any nucleic acid sequence.

10. Method according to any one of claims 1 to 9, wherein at least one second control nucleic acid sequence that binds to nucleic acid sequences of all microorganisms is additionally added to the sample in step c).

11. Method according to any one of claims 1 to 10, wherein the detectable label is a luminescent label, in particular a fluorescent label, or wherein the detectable label is a reporter enzyme, preferably selected from the group consisting of tyrosinase, peroxidase, sulfite oxidase, alkaline phosphatase, glucose oxydase, guanine oxidase.

## Patentansprüche

1. Verfahren zum Nachweisen von mindestens einem spezifischen Mikroorganismus in einer Probe, wobei zumindest eine Sonden-Nukleinsäuresequenz geeignet ist mit mindestens einer Ziel-Nukleinsäuresequenz dieses Mikroorganismus' zu hybridisieren, diese Sonden-Nukleinsäuresequenz umfasst mindestens einen nachweisbaren Marker, der zum Emittieren mindestens eines nachweisbaren Signals geeignet ist, wobei das nachweisbare Signal einen ersten Wert annimmt, wenn die Probensequenz nicht an die Zielsequenz gebunden ist, und einen zweiten Wert, wenn die Probensequenz an die Zielsequenz gebunden ist, wobei der zweite Wert des nachweisbaren Signals im Vergleich zum ersten Wert des nachweisbaren Signals verringert, erhöht oder verändert ist, dieses Verfahren umfasst:
a) In einer bereitgestellten Probe, in welcher der Mikroorganismus vermutlich enthalten ist;
b) Perforieren oder Lysieren des Mikroorganismus' innerhalb der Probe;
c) Zugeben der Sonden-Nukleinsäuresequenz in die Probe unter Bedingungen, welche eine *in-vitro* - Hybridisierung der Probensequenz mit der Zielsequenz ermöglichen, wobei diese Bedingungen ein zumindest zeitweises Erhitzen der Probe auf eine vorbestimmte Temperatur oberhalb der Raumtemperatur und anschließend ein Abkühlen der Probe auf eine geringere Temperatur umfasst;
d) Kontinuierliches Messen von Werten des nachweisbaren Signals der Sonden-Nukleinsäuresequenz in der Probe zu einer Vielzahl von Messzeitpunkten im Verlauf der signalerzeugenden Reaktion, umfassend das Messen von Werten des nachweisbaren Signals während und/oder nach dem Erhitzen und während und/oder nach dem Abkühlen; und
e) Kontinuierliches Analysieren der gemessenen Werte des nachweisbaren Signals der Sonden-Nukleinsäuresequenz, wobei angezeigt wird, dass die Probe den Mikroorganismus enthält, wenn die gemessenen Werte dem zweiten Wert des nachweisbaren Signals entsprechen.

2. Verfahren nach Anspruch 1, wobei die Messung beendet wird, sobald die Analyse in Schritt e) ergibt, dass die Probe den Mikroorganismus enthält.

3. Verfahren nach Anspruch 1 oder 2, wobei zumindest die Schritte d) und e) automatisch ausgeführt und die gemessenen Werte elektronisch ausgelesen und digital verarbeitet werden.

4. Verfahren nach einem der Ansprüche 1 bis 3, wobei die Temperatur nach Schritt c) für eine vorbestimmte Zeitdauer konstant gehalten wird.

5. Verfahren nach Anspruch 4, wobei mindestens ein Wert des nachweisbaren Signals in der Probe gemessen wird, während die Temperatur konstant gehalten wird.

6. Verfahren nach einem der Ansprüche 1 bis 5, wobei die Sonden-Nukleinsäuresequenz eine lineare Nukleinsäure oder ein molekulares Beacon ist.

7. Verfahren nach einem der Ansprüche 1 bis 6, wobei die Ziel-Nukleinsäuresequenz eine DNA-Sequenz oder eine RNA-Sequenz, insbesondere eine rRNA-Sequenz, ist.

8. Verfahren nach einem der Ansprüche 1 bis 7, wobei in Schritt c) der Probe zusätzlich mindestens eine erste Kontroll-Nukleinsäuresequenz zugegeben wird, Werte eines nachweisbaren Signals der ersten Kontroll-Nukleinsäuresequenz werden an den Messpunkten in Schritt d) gemessen, und wobei die Analyse in Schritt e) einen Vergleich der gemessenen Werte des nachweisbaren Signals der Sonden-Nukleinsäuresequenz mit den gemessenen Werte des nachweisbaren Signals der ersten Kontroll-Nukleinsäuresequenz umfasst, wobei angezeigt wird, dass die Probe den Mikroorganismus enthält, wenn der für die Sonden-Nukleinsäuresequenz gemessene Wert sich an jedem Messpunkt von dem entsprechenden, für die erste Kontroll-Nukleinsäuresequenz gemessenen Wert unterscheidet und der Unterschied zwischen den verglichenen Werten einen vorbestimmten Schwellenwert überschreitet.

9. Verfahren nach Anspruch 8, wobei die erste Kontroll-Nukleinsäuresequenz eine Sequenz ist, die an keine Nukleinsäuresequenz bindet.

10. Verfahren nach einem der Ansprüche 1 bis 9, wobei mindestens eine zweite Kontroll-Nukleinsäuresequenz, welche an Nukleinsäuresequenzen aller Mikroorganismen bindet, der Probe in Schritt c) zusätzlich zugegeben wird.

11. Verfahren nach einem der Ansprüche 1 bis 10, wobei der nachweisbare Marker ein Lumineszenz-Marker ist, insbesondere ein Fluoreszenz-Marker, oder wobei der nachweisbare Marker ein Reporter-Enzym ist, vorzugsweise ausgewählt aus der Gruppe bestehend aus Tyrosinase, Peroxidase, Sulfitoxidase, alkalische Phosphatase, Glukose-Oxydase, Guanin-Oxidase.

## Revendications

1. Procédé de détection d'au moins un micro-organisme spécifique dans un échantillon, dans lequel au moins une séquence sonde d'acides nucléiques est capable de s'hybrider avec au moins une séquence cible d'acides nucléiques dudit micro-organisme, ladite séquence sonde d'acides nucléiques comprenant au moins un marqueur pouvant être détecté qui est capable d'émettre au moins un signal pouvant être détecté, dans lequel le signal pouvant être détecté prend une première valeur lorsque la séquence sonde n'est pas liée à la séquence cible et une seconde valeur lorsque la séquence sonde est liée à la séquence, dans lequel la seconde valeur du signal pouvant être détecté diminue, augmente ou change par rapport à la première valeur du signal pouvant être détecté, ledit procédé comprenant:
a) dans un échantillon fourni supposé inclure le micro-organisme ;
b) perforer ou lyser les micro-organismes situés à l'intérieur de l'échantillon ;
c) ajouter la séquence sonde d'acides nucléiques à l'échantillon dans des conditions qui permettent une hybridation *in vitro* de la séquence sonde avec la séquence cible, dans lequel lesdites conditions comprennent le chauffage de l'échantillon au moins de manière temporaire à une température prédéterminée au-dessus de la température ambiante et ensuite le refroidissement de l'échantillon à une température inférieure ;
d) mesurer de manière continue les valeurs du signal pouvant être détecté pour la séquence sonde d'acides nucléiques dans l'échantillon au niveau d'une pluralité de points de mesure dans le temps pendant le cours de la réaction produisant un signal, comprenant des valeurs de mesure du signal pouvant être détecté pendant et/ou après le chauffage et pendant et/ou après le refroidissement ; et
e) analyser de manière continue les valeurs mesurées du signal pouvant être détecté de la séquence d'acides nucléiques, dans lequel il est indiqué que l'échantillon contient le micro-organisme si les valeurs mesurées correspondent à la seconde valeur du signal pouvant être détecté.

2. Procédé selon la revendication 1, dans lequel la mesure est arrêtée une fois que l'analyse dans l'étape e) indique que l'échantillon contient le micro-organisme.

3. Procédé selon la revendication 1 ou la revendication 2, dans lequel les étapes d) et e) sont au moins effectuées de manière automatique, et les valeurs mesurées sont lues de manière électronique et traitées de manière digitale.

4. Procédé selon l'une quelconque des revendications 1 à 3, dans lequel après l'étape c), on maintient la température constante pendant une période de temps prédéterminée.

5. Procédé selon la revendication 4, dans lequel une valeur du signal pouvant être détecté dans l'échantillon est au moins mesurée pendant qu'on maintient la température constante.

6. Procédé selon l'une quelconque des revendications 1 à 5, dans lequel la séquence sonde d'acides nucléiques est un acide nucléique linéaire ou une balise moléculaire.

7. Procédé selon l'une quelconque des revendications 1 à 6, dans lequel la séquence cible d'acides nucléiques est une séquence d'ADN ou une séquence d'ARN, en particulier une séquence d'ARNr.

8. Procédé selon l'une quelconque des revendications 1 à 7, dans lequel dans l'étape c) une première séquence d'acides nucléiques de contrôle est de plus au moins ajoutée à l'échantillon, les valeurs d'un signal pouvant être détecté pour la première séquence d'acides nucléiques de contrôle étant mesurées aux points de mesure dans l'étape d), et dans lequel l'analyse dans l'étape e) comprend une comparaison des valeurs mesurées du signal pouvant être détecté de la séquence sonde d'acides nucléiques avec les valeurs mesurées du signal pouvant être détecté de la première séquence d'acides nucléiques de contrôle, dans lequel il est indiqué que l'échantillon contient le micro-organisme si la valeur mesurée pour la séquence sonde d'acides nucléiques à n'importe quel point de mesure est différente de la valeur mesurée respective pour la première séquence d'acides nucléiques de contrôle et la différence entre les valeurs comparées dépasse un seuil prédéterminé.

9. Procédé selon la revendication 8, dans lequel la première séquence d'acides nucléiques de contrôle est une séquence qui ne se lie à aucune séquence d'acides nucléiques.

10. Procédé selon l'une quelconque des revendications 1 à 9, dans lequel une seconde séquence d'acides nucléiques de contrôle qui se lie à des séquences d'acides nucléiques de tous les micro-organismes est de plus en outre ajoutée à l'échantillon dans l'étape c).

11. Procédé selon l'une quelconque des revendications 1 à 10, dans lequel le marqueur pouvant être détecté est un marqueur luminescent, en particulier un marqueur fluorescent, ou dans lequel le marqueur pouvant être détecté est une enzyme rapporteur, de préférence choisie dans le groupe constitué de la tyrosinase, de la peroxydase, de la sulfite oxydase, de la phosphatase alcaline, de la glucose oxydase et de la guanine oxydase.
